# EUROPEAN PATENT SPECIFICATION

(11) **EP 2 393 478 B1**
(45) Date of publication and mention of the grant of the patent: **17.06.2015**
(21) Application number: 10706802.5
(22) Date of filing: 02.02.2010
(51) Int. Cl.: A61K 9/00, A61K 9/20, A61K 31/198

(54) **TASTE AND ODOR MASKED PHARMACEUTICAL COMPOSITIONS WITH HIGH BIOAVAILABILITY**
PHARMAZEUTISCHE ZUSAMMENSETZUNG MIT HOHER BIOVERFÜGBARKEIT UND MASKIERTEM GESCHMACK UND GERUCH
COMPOSITIONS PHARMACEUTIQUES À L'ODEUR ET AU GOÛT MASQUÉS HAUTEMENT BIODISPONIBLES

(30) Priority: 05.02.2009 TR 200900882
(43) Date of publication of application: 14.12.2011
(73) Proprietor: Bilgic, Mahmut, 34220 Esenler-Istanbul (TR)
(72) Inventor: Bilgic, Mahmut, 34220 Esenler-Istanbul (TR)
(86) International application number: PCT/TR2010/000022
(87) International publication number: WO 2010/090611

(56) References cited:
- EP-B1- 0 481 294
- WO-A1-2008/024020
- DE-A1- 4 103 360
- GB-A- 2 192 790
- US-A- 4 092 410
- US-A1- 2008 299 224
- "Rote Liste 2004", 1 January 2004 (2004-01-01), Rote Liste Service GmbH, Germany, XP002613304, ISBN: 3-87193-286-8 pages 24113-24138, Chapter 24113-24138; especially 24113,24131,24136

## Description

### Field of the invention

The present invention is related to the taste and odor masked pharmaceutical compositions which have high bioavailability and their medical use.

### Background of the invention

The present invention provides a composition which is effective in the treatment of acute and chronic respiratory diseases and bronchial secretion disorders for removal and reduction of thick and viscous mucous and in cases where expectoration should be facilitated and for prevention of liver failure caused by high dose paracetamol intake. The effect caused by the composition prepared according to the present invention is hereinafter referred to as "desired effect".

The composition disclosed herein includes NAC as a mucolytic agent at a ratio up to 70% by weight. This ratio includes 900-2500 mg dose range which is higher than the ratio of conventional NAC treatment.

NAC (formula I) is an acetylated derivative of the aminoacid L-cysteine which has a free thiol group (N-Acetyl-L-Cysteine).

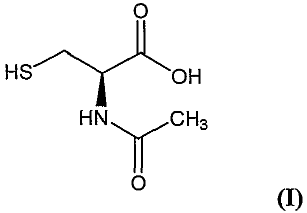

NAC was is described in patent US3184505 A. The patent discloses processes for the preparation of NAC, its mucolytic activity and its use in the treatment of respiratory diseases.

NAC, which is the ready to use form of L-cysteine is more stable than L-cysteine and its absorption is higher than the absorption of L-cysteine. NAC has antioxidant activity as a reducing agent. Moreover, in addition to its liver protective and mucolytic activity, it has anti-apoptotic activity. Mucolytic activity of NAC is assumed to be related to the thiol groups' ability of reducing the disulphide bonds of the mucoproteins that are present in the mucous. With this effect; the viscosity of bronchial secretation is reduced and taking them out by cough reflex becomes easier. As a result breathing becomes easier. Moreover, NAC accelerates detoxication by the conversion into metabolites which are capable of stimulating glutation synthesis in the body and shows free radical scavenging effect. It is assumed that; NAC and glutathione's reduction of inflammation by neutralizing endogenous and exogenous oxidants is part of the mechanism of action.

The present invention includes NAC compositions which is effective in the treatment of acute and chronic respiratory diseases and bronchial secretion disorders for removal and reduction of thick and viscous mucous and in cases where expectoration should be facilitated and for prevention of liver failure caused by high dose paracetamol intake.

Object of the present invention is to provide a composition prepared according to the present invention that comprise pharmaceutically acceptable, non-toxic and therapeutically effective amounts of NAC and to formulate said active agent.

However there remains some problems in the pharmaceutical compositions containing NAC. The first one is bioavailability problem. When NAC is taken orally, it reaches liver by portal cirulation, after being rapidly absorbed from the gastrointestinal tract, NAC undergoes an extensive first-pass metabolism. First-pass effect occurs in the small intestine and liver cells. Depending on the first-pass metabolism, NAC has low bioavailability (approximately 10%). This case results in a great reduction in the efficiency of the drug. Therefore, pharmaceutical formulations which increase the bioavailability of NAC are needed. However, in the meantime the things that must be kept in mind increases. For example, since NAC is a highly acidic substance when it remains in the mouth for a long time, it causes irritation of the oral cavity and mucous membrane of the tongue. Moreover, due to the presence of sulphur group in the molecule, taste and odor of NAC is extremely bad.

In the patent numbered US5080906, it is indicated that NAC salts such as alkali metal or basic amino acid salts have been employed in order to increase the bioavailability of NAC which is taken orally but they were not found to be effective. In the same patent, a method of combination comprising NAC and its (hydoxymethyl)aminomethane or a pharmaceutically acceptable salt in the molar ratio between 1:0.8 and 1:1.2 are explained as a solution to increase bioavailability. However, no solution related to masking the unpleasent taste and odor of NAC was disclosed in this patent.

In patent numbered EP0339508 B1, a solution is disclosed for prevention of irritation of mucuous membrane and to mask the taste of NAC. The patent comprises a mouth soluble tablet composition wherein, the weight ratio of sodium bicarbonate and potassium bicarbonate is between 93:7 and 97:3 and wherein the said mixture is present in an amount of 45-55 parts per weight based on 100 parts by weight of acetylcysteine; a mixture of two carbohydrates selected from the group consisting of sorbitol xylitol and fructore, wherein the weight ratio between said two carbohydrates is from 0.8 to 1.2 and 1.2 to 0.8, and wherein said carbohydrate mixture is present in an amount of 20-50 parts by weight based on 1 part by weight of acetylcysteine and a fruit flavoring agent.

In EP0340662 B1, a water soluble pharmaceutical formulation comprising 5-25 parts by weight NAC, 70-85 parts xylitol, 0.5-1.2 parts sodium saccharin bihydrate, 0.4-1.2 part beta carotene and 4 - 7.5 parts flavoring agent is disclosed as a solution to mask the unpleasent taste and odor of NAC.

In patent numbered EP0481294 B2, orally administered, fast dissolving, solid drug preparations have been described as a solution to mask the unpleasent taste and odor of NAC. Said drug preparations consist of; NAC whose proportion by the total weight of formulation is at least 50%, and the remaining amount of the preparation contains as inactive ingredients in relation to NAC by weight 15-25% cellulose and/or 4-12% cellulose derivatives, 25-35% sugar alcohol, 4-12% sweetener, 2-8%, flavoring and, if need be, 2-8% adjuvant that contain other auxiliary agent.

As a result, in state of the art, the unpleasent taste and odor and its high rate of acidity is usually masked with use of sweeteners and flavoring agents in high amounts. But this time, high amounts of inactive substances cause the drug to be in high weight formulation. This situation interferes with use of NAC in high amounts in order to increase the bioavailability. Using high amount of NAC makes the taste and odor of the formulation worse. As a result, there is a strong need for pharmaceutical compositions which allows to use NAC in high amounts in order to increase the bioavailability and to mask the taste and odor without using sweetener and flavoring agent in high amounts.

The present invention provides taste and odor masked pharmaceutical compositions with high bioavailability in which NAC shows "desired effect". Pharmaceutical compositions prepared according to the present invention include NAC in high amounts to increase the bioavailability and sodium chloride as a taste regulator to mask the unpleasent taste and odor of NAC. Additionally, the suitable pharmaceutical compositions of the invention include at least one sweetener and/or flavoring agent at low rates.

### Summary of the invention

The present invention relates to a pharmaceutical composition characterized in that the composition comprises, with respect to the core weight of the composition, NAC in the range of 900-2500 mg, sodium chloride at a rate up to 5% by weight, at least one sweetener and/ or flavoring agent at a rate up to 5% by weight and, optionally one or more pharmaceutically acceptable excipients selected from stabilizing agent, diluent, binder, disintegrant, lubricant, glidant and surfactant, wherein said composition is in the dosage forms of tablet, capsule, fast dissolving tablet or effervescent tablet.

The present invention is further related to a pharmaceutical composition used in the manufacture of a drug which is effective in the treatment of acute and chronic respiratory diseases and bronchial secretion disorders; for removal and reduction of thick and viscous mucous; in cases where expectoration should be facilitated and for prevention of liver failure caused by high dose paracetamol intake characterized in that the said pharmaceutical composition comprises NAC at a ratio up to 70% by weight and sodium chloride as a taste regulator.

### Detailed description of the invention

The present invention provides taste and odor masked pharmaceutical compositions which also have high bioavailability wherein the composition has desired effect of NAC.

As a result of studies, the present inventors have surprisingly found that using NAC as the active ingredient in an amount up to 70% by weight, increases bioavailability and thus optimum activity (efficiency) is provided for the prevention and treatment of various diseases and conditions; using sodium chloride as a taste regulator instead of using sweetener and flavoring agents in high amounts masks the unpleasent taste and odor of NAC.

Another characteristic of the invention is that the formulation comprising NAC in an amount up to 70% by weight, sodium chloride in a sufficient amount, at least one sweetener and/or flavoring agent in sufficient amount and optionally pharmaceutically acceptable one or more excipients selected from stabilizing agent, diluent, binder, disintegrant, lubricant, glidant and surfactant provides optimum efficiency for the treatment and prevention of various diseases and also masks the unpleasent taste and odor of NAC.

The ratio described with the term "in an amount up to 70% by weight" means the dose range 900-2500 mg which is higher than the ratio of conventional NAC treatment; and which is preferred to solve the bioavailability problem of NAC in order to provide the desired therapeutic activity.

Here, the term "several diseases and conditions" is related to; acute and chronic diseases of the respiratory diseases, bronchial secretion disorders, liver failure caused by high dose paracetamol intake, removal and reduction of thick and viscous mucous and facilitation of expectoration.

The terms "sufficient amount" and "optionally" mean that using sodium chloride up to 5% by weight, sweetener and/or flavoring agent up to 5% by weight and one or more pharmaceutically acceptable excipients to be used when necessary which are selected from a group consisting of diluent, binder, disintegrant, lubricant, glidant, surface active agent, sweetener and flavoring agent.

Pharmaceutically acceptable sweeteners can be selected from sucralose, sucrose, fructose, glucose, galactose, xylose, dextrose, levulose, lactose, maltose, maltodextrin, mannitol, maltitol, maltol, sorbitol, xylitol, erythritol, laktitol, isomalt, corn syrup, saccharin, saccharin salts, acesulfame potassium, aspartame, D-tryptophan, monoammonium glycerssinate, neohesperidin dihydrochalcone, thaumatin, neotame, alitam, stevioside and cyclamate.

Pharmaceutically acceptable flavoring agents can be selected from natural aroma oil (e.g. peppermint oil, partridge currant oil, clove bud oil, parsley oil, eucalyptus oil, lemon oil, orange oil, etc.), menthol, mentane, anethole, methyl salicylate, eucalyptol, cinnamon, 1-methyl acetate, salvia, eugenol, oxanone, alpha-ionone, marjoram, lemon, orange, propenyl guaethol acetyl, sinnamon, vanilla, thymol, linaolol, cinnamaldehyde glycerol acetal, N-substituted p-menthane-3-carboxyamide, 3,1- methoxy propane 1,2-diol.

Pharmaceutically acceptable diluents can be selected from lactose, microcrystalline cellulose, starch, prejelatinized starch, modified starch, calcium phosphate (dibasic and/or tribasic), calcium sulfate trihydrate, calcium sulfate dihydrate, calcium carbonate, kaolin, lactitol, powdered cellulose, dextrose, dextrates, dextrin, sucrose, maltose, fructose, mannitol, sorbitol and xylitol. Diluents can be present in the range of preferably 0-85%, more preferably 0.1-75% by weight.

Pharmaceutically acceptable binders can be selected from starch (e.g.potato starch, corn starch, wheat starch, etc.), sucrose, glucose, dextrose, lactose, sugars such as maltodexstrin, natural and synthetic gums (such as acacia), gelatin, cellulose derivatives (microcrystalline cellulose, HPC, HEC, HPMC, carboxymethylcellulose, methylcellulose, ethylcellulose, etc.), polyvinylpyrrolidone (PVP), polyethylene glycol (PEG), waxes, calcium carbonate, calcium phosphate, alcohols (e.g. sorbitol, xylitol, mannitol) and water. Binder can be present in the range of preferably 0-10%, more preferably 0,1-5% by weight.

Pharmaceutically acceptable disintegrants can be selected from starch (corn starch, potato starch), sodium starch glycolate, pregelatinized starch, cellulose derivatives (e.g. croscarmellose sodium or microcrystalline cellulose), polyvinylpyrrolidone (PVP), crospovidone, alginic acid, sodium alginate, clays (xanthan gum or veegum etc.), ion-exchange resins and effervescent systems (alkali or alkaline earth metal carbonates [sodium carbonate, sodium hydrogen carbonate, potassium carbonate, potassium hydrogen carbonate, calcium carbonate, etc.]; water soluble polybasic organic acids and salts thereof such as sodium hydrogen sulfate, potassium hydrogen sulfate, sodium dihydrogen phosphate, succinic acid, tartaric acid, adipic acid, citric acid). Disintegrants can be present in the range of preferably 0-85%, more preferably 0.1-75% by weight.

Pharmaceutically acceptable lubricants can be selected from metallic stearates (magnesium stearate, calcium stearate, aluminum stearate, etc.), fatty acid esters (e.g. sodium stearyl fumarate), fatty acids (e.g. stearic acid), fatty alcohols, glyceryl behenate, mineral oil, paraffines, hydrogenated vegetable oils, leucine, polyethylene glycols (PEG), metallic lauryl sulfates (sodium lauryl sulfate, magnesium lauryl sulfate, etc.), sodium chloride, sodium benzoate, sodium acetate and talc. Lubricants can be present in the range of preferably 0-10%, more preferably 0.1-5% by weight.

Pharmaceutically acceptable glidants can be selected from silicon dioxide, magnesium trisilicate, powdered cellulose, starch, talc, tribasic calcium phosphate, metallic stearates, calcium silicate and metallic lauryl sulfate. Glidant can be present in the pharmaceutical formulation in proportions less than 1% by weight.

Pharmaceutically acceptable surfactants can be selected from polyoxyethlene-sorbitan-fatty acid esters (polysorbates), sodium lauryl sulfate, sodium stearyl fumarate, polyoxyethylene alkyl ethers, sorbitan fatty acid esters, polyethylene glycols (PEG), polyoxyethylene hint oil derivatives, docusate sodium, quaternary ammonium compounds, amino acids such as L-leucine, sugar esters of fatty acids and glycerides of fatty acids. Surfactant can be present in the range of preferably 0-10%, more preferably 0.1-5% by weight.

Additionally, pharmaceutically acceptable other excipients such as solubility modulators, electrolytes, colorants and coatings can be present in the formulation.

Pharmaceutical acceptable dosage forms are tablet, capsule, fast dissolving tablet, effervescent tablet, effervescent granule, fast dissolving granule, fast dissolving powder mixture, or dry powder form to prepare syrup.

Examples of pharmaceutical formulations of the invention are given below. These examples are given to illustrate the invention; the invention is not limited to disclosed examples.

### EXAMPLES

### Example 1.

| **Content** | **Amount (mg)** |
|---|---|
| NAC | 900 |
| Sodium Chloride | 50 |
| Citric acid anhydride | 1140 |
| Sodium hydrogen carbonate | 775 |
| Aspartame | 30 |
| Lemon flavor | 20 |
| PEG 6000 | 25 |
| PVP K-30 | 60 |
| **Tablet weight** | 3000 |

### Example 2.

| **Content** | **Amount (mg)** |
|---|---|
| NAC | 1200 |
| Sodium Chloride | 60 |
| Citric acid anhydride | 1035 |
| Sodium hydrogen carbonate | 770 |
| Aspartame | 30 |
| Lemon flavor | 20 |
| PEG 6000 | 25 |
| PVP K-30 | 60 |
| **Tablet weight** | 3200 |

### Example 3.

| **Content** | **Amount (mg)** |
|---|---|
| NAC | 2500 |
| Sodium Chloride | 75 |
| Citric acid anhydride | 920 |
| Sodium hydrogen carbonate | 730 |
| Aspartame | 40 |
| Lemon flavor | 30 |
| PEG 6000 | 30 |
| PVP K-30 | 75 |
| **Tablet weight** | 4400 |

## Claims

1. A pharmaceutical composition **characterized in that** the composition comprises, with respect to the core weight of the composition,
• NAC in the range of 900-2500 mg
• Sodium chloride at a rate up to 5% by weight
• At least one sweetener and/or flavoring agent at a rate up to 5% by weight and
• Optionally one or more pharmaceutically acceptable excipients selected from stabilizing agent, diluent, binder, disintegrant, lubricant, glidant and surfactant,
wherein said composition is in the dosage forms of tablet, capsule, fast dissolving tablet or effervescent tablet.

2. The composition according to claim 1 **characterized in that**, sweeteners are selected from sucralose, sucrose, fructose, glucose, galactose, xylose, dextrose, levulose, lactose, maltose, maltodextrin, mannitol, maltitol, maltol, sorbitol, xylitol, erythritol, lactitol, isomalt, corn syrup, saccharin, saccharin salts, asesulfame potassium, aspartame, D-tryptophan, monoammonium glyserrisate, neohesperidin dihydrochalcone, thaumatin, neotame, alitam, stevioside and cyclamate.

3. The composition according to claim 1 **characterized in that**, flavoring agents are selected from natural aroma oils (e.g. peppermint oil, Partridge currant oil, clove bud oil, parsley oil, eucalyptus oil, lemon oil, orange oil, etc.), menthol, menta, anethole, methyl salicylate, eucalyptol, cinnamon, 1-methyl acetate, salvia, eugenol, oxanone, alpha-irison, marjoram, lemon, orange, propenyl guaethol acetyl, sinnamon, vanilla, thymol, linaolol, cinnamaldehyde glycerol acetal, N-substituted-p-menthane-3-carboxyamide, 3,1- methoxy propane 1,2-diol.

4. The composition according to claim 1, **characterized in that** diluent is selected from lactose, microcrystalline cellulose, starch, prejelatinized starch, modified starch, calcium phosphate (dibasic and/or tribasic), calcium sulfate trihydrate, calcium sulfate dihydrate, calcium carbonate, kaolin, lactitol, powdered cellulose, dextrose, dextrates, dextrin, sucrose, maltose, fructose, mannitol, sorbitol and xylitol.

5. The composition according to the claim 4 **characterized in that** diluent is in the range of preferably 0-85%, more preferably 0.1-75% by weight.

6. The composition acorrding to claim 1 **characterized in that** binder is selected from starch (e.g.potato starch, corn starch, wheat starch, etc.), sucrose, glucose, dextrose, lactose, sugars such as maltodextrin, natural and synthetic gums (such as acacia), gelatin, cellulose derivatives (microcrystalline cellulose, HPC, HEC, HPMC, carboxymethylcellulose, methylcellulose, ethylcellulose, etc.), polyvinylpyrrolidone (PVP), polyethylene glycol (PEG), waxes, calcium carbonate, calcium phosphate, alcohols (e.g. sorbitol, xylitol, mannitol) and water.

7. The composition according to the claim 6 **characterized in that** binder is in the range of preferably 0-10%, more preferably 0,1-5% by weight.

8. The composition according to claim 1 **characterized in that**, disintegrants selected from starch (corn starch, potato starch), sodium starch glycolate, pregelatinized starch, cellulose derivatives (e.g. croscarmellose sodium or microcrystalline cellulose), polyvinylpyrrolidone (PVP), crospovidone, alginic acid, sodium alginate, clays (xanthan gum or veegum etc.), ion-exchange resins and effervescent systems (alkali or alkaline earth metal carbonates [sodium carbonate, sodium hydrogen carbonate, potassium carbonate, potassium hydrogen carbonate, calcium carbonate, etc.], water soluble polybasic organic acids and salts thereof such as sodium hydrogen sulfate, potassium hydrogen sulfate, sodium dihydrogen phosphate, succinic acid, tartaric acid, adipic acid, citric acid.

9. The composition according to the claim 8 **characterized in that** disintegrant is in the range of preferably 0-85%, more preferably 0.1-75% by weight.

10. The composition according to claim 1 **characterized in that** lubricant is selected from metallic stearates (magnesium stearate, calcium stearate, aluminum stearate, etc.), fatty acid esters (e.g. sodium stearyl fumarate), fatty acids (e.g. stearic acid), fatty alcohols, glyceryl behenate, mineral oil, paraffines, hydrogenated vegetable oils, leucine, polyethylene glycols (PEG), metallic lauryl sulfates (sodium lauryl sulfate, magnesium lauryl sulfate, etc.), sodium chloride, sodium benzoate, sodium acetate and talc.

11. The composition according to the claim 10 **characterized in that** lubricant is in the range of preferably 0-10%, more preferably 0.1-5% by weight.

12. The composition according to claim 1 **characterized in that** glidant is selected from silicon dioxide, magnesium trisilicate, powdered cellulose, starch, talc, tribasic calcium phosphate, metallic stearates, calcium silicate and metallic lauryl sulfate.

13. The composition according to the claim 12 **characterized in that** glidant is less than 1% by weight.

14. The composition according to claim 1 **characterized in that** surfactant is selected from polyoxyethlene-sorbitan-fatty acid esters (polysorbates), sodium lauryl sulfate, sodium stearyl fumarate, polyoxyethylene alkyl ethers, sorbitan fatty acid esters, polyethylene glycols (PEG), polyoxyethylene hint oil derivatives, docusate sodium, quaternary ammonium compounds, amino acids such as L-leucine, sugar esters of fatty acids and glycerides of fatty acids, and is present in the range of preferably 0-10%, more preferably 0.1-5% by weight.

15. The composition according to the any preceeding claims **characterized in that** dosage form comprises a pharmaceutical composition given below;
NAC at a ratio up to 70% by weight
Sodium chloride at a ratio up to 5% by weight
Citric acid anhydride at a ratio 0-85% by weight
Sodium hydrogen carbonate at a ratio 0-85% by weight
Aspartame and/or lemon flavor at a ratio up to 5% by weight
PEG at a ratio 0-10% by weight; and
PVP at a ratio 0-10% by weight.

## Patentansprüche

1. Pharmazeutische Zusammensetzung, **dadurch gekennzeichnet, dass** die Zusammensetzung, bezogen auf das Kerngewicht der Zusammensetzung, die folgenden Elementen beinhaltet:
• NAC im Bereich von 900-2500 mg
• Natriumchlorid in einer Mengebis zu 5% nach Gewicht
• Mindestens ein Süßungsmittel und/oder Aromastoff in einer Menge bis zu 5% nach Gewicht und
• gegebenenfalls einen oder mehrere pharmazeutisch annehmbare Hilfsstoffe, die aus Stabilisierungsmittel, Verdünnungsmittel, Bindemittel, Sprengmittel, Gleitmittel, Gleitstoffen und Tensid ausgewählt werden, wobei die erwähnte Zusammensetzung in den Dosierungsformen von Tabletten, Kapseln, schnell löslichen Tabletten oder Brausetabletten ist.

2. Die Zusammensetzung nach Anspruch 1, **dadurch gekennzeichnet, dass** Süßstoffe aus Sucralose, Saccharose, Fructose, Glucose, Galactose, Xylose, Dextrose, Lävulose, Lactose, Maltose, Maltodextrin, Mannit, Maltit, Maltol, Sorbitol, Xylitol, Erythritol, Lactitol, Isomalt, Maissirup, Saccharin, Saccharin-Salze, Acesulfam-Kalium, Aspartam, D-Tryptophan, Monoammonium- Glyserrisate, Neohesperidin-Dihydrochalcon, Thaumatin, Neotam, Alitam, Steviosid und Cyclamat ausgewählt werden.

3. Die Zusammensetzung nach Anspruch 1, **dadurch gekennzeichnet, dass** Aromastoffe aus natürlichen Aromaöle (z.B. Pfefferminzöl, Partridge Johannisbeere Öl, Nelkenknospenöl, Petersilienöl , Eukalyptusöl , Zitronenöl, Orangenöl, usw.), Menthol, Menta, Anethol, Methylsalicylat, Eucalyptol, Zimt, 1-Methyl-Azetat, Salbei, Eugenol, Oxanone, Alpha-Irison, Majoran, Zitrone, Orange, Propenyl guaethol Acetyl, Sinnamon, Vanille, Thymol, Linalool, Zimtaldehyd Glycerin Acetal, N-substituierte -p-menthan-3-Carbonsäureamide-3,1- Methoxypropan-1,2-diol ausgewählt werden.

4. Die Zusammensetzung nach Anspruch 1, **dadurch gekennzeichnet, dass** Verdünnungsmittel aus Lactose, mikrokristalliner Cellulose, Stärke, vorgelatinierter Stärke, modifizierter Stärke, Calciumphosphat (dibasisches und/oder dreibasisches), Calciumsulfat-Trihydrat, Calciumsulfat-Dihydrat, Calciumcarbonat, Kaolin, Lactit, pulverisierter Cellulose, Dextrose, Dextrate, Dextrin, Saccharose, Maltose, Fructose, Mannit, Sorbit und Xylit ausgewählt werden.

5. Die Zusammensetzung nach Anspruch 4, **dadurch gekennzeichnet, dass** Verdünnungsmittel im Bereich von vorzugsweise 0-85%, besser 0,1-75% nach Gewicht liegt.

6. Die Zusammensetzung nach Anspruch 1, **dadurch gekennzeichnet, dass** Bindemittel aus Stärke (z.B. Kartoffelstärke, Maisstärke, Weizenstärke, usw.), Saccharose, Glucose, Dextrose, Lactose, Zucker wie Maltodextrin, natürliche und synthetische Gummis (wie Akazien), Gelatine, Cellulosederivaten (mikrokristalline Cellulose, HPC, HEC, HPMC, Carboxymethylcellulose, Methylcellulose, Ethylcellulose, usw.), Polyvinylpyrrolidon (PVP), Polyethylenglykol (PEG), Wachse, Calciumcarbonat, Calciumphosphat, Alkohole (z.B. Sorbitol, Xylit, Mannit) und Wasser ausgewählt wird.

7. Die Zusammensetzung nach Anspruch 6, **dadurch gekennzeichnet, dass** Bindemittel im Bereich von vorzugsweise 0-10%, besser 0,1-5% nach Gewicht liegt.

8. Die Zusammensetzung nach Anspruch 1, **dadurch gekennzeichnet, dass** Sprengmittel aus Stärke (Maisstärke, Kartoffelstärke), Natriumstärkeglycolat, vorgelatinisierter Stärke, Cellulosederivaten (z.B. Croscarmellose-Natrium oder mikrokristalline Cellulose), Polyvinylpyrrolidon (PVP), Crospovidone, Alginsäure, Natriumalginat, Lehme (Xanthangummi oder Veegum usw.), Ionenaustauschharze und Brausesysteme (Alkali- oder Erdalkalicarbonate [Natriumcarbonat, Natriumhydrogencarbonat, Kaliumcarbonat, Kaliumhydrogencarbonat, Calciumcarbonat, usw.], Wasser lösliche polybasische organische Säuren und deren Salze wie Natriumhydrogensulfat, Kaliumhydrogensulfat, Natriumdihydrogenphosphat, Bernsteinsäure, Weinsäure, Adipinsäure, Zitronensäure gewählt werden.

9. Die Zusammensetzung nach Anspruch 8, **dadurch gekennzeichnet, dass** das Sprengmittel im Bereich von vorzugsweise 0-85%, besser 0,1-75% nach Gewicht liegt.

10. Die Zusammensetzung nach Anspruch 1, **dadurch gekennzeichnet, dass** Gleitmittel aus Metallstearaten (Magnesiumstearat, Calciumstearat, Aluminiumstearat, usw.), Fettsäureester (z.B. Natriumstearylfumarat), Fettsäuren (z.B. Stearinsäure), Fettalkohole, Glycerylbehenat, Mineralöl, Paraffin, hydrierten pflanzlichen Ölen, Leucin, Polyethylenglykole (PEG), metallischen Laurylsulfaten (Natriumlaurylsulfat, Magnesiumlaurylsulfat, usw.), Natriumchlorid, Natriumbenzoat, Natriumacetat und Talk gewählt wird.

11. Die Zusammensetzung nach Anspruch 10, **dadurch gekennzeichnet, dass** Gleitmittel im Bereich von vorzugsweise 0-10%, besser 0,1-5 % nach Gewicht liegt.

12. Die Zusammensetzung nach Anspruch 1, **dadurch gekennzeichnet, dass** Fließregulierungsmittelaus Siliziumdioxid, Magnesiumtrisilikat, pulverisierter Cellulose, Stärke, Talk, Tricalciumphosphat, Metallstearaten, Calciumsilikat und Metall Laurylsulfat ausgewählt wird.

13. Die Zusammensetzung nach Anspruch 12, **dadurch gekennzeichnet, dass** Fließregulierungsmittelweniger als 1 % nach Gewicht ist.

14. Die Zusammensetzung nach Anspruch 1, **dadurch gekennzeichnet, dass** Tensid aus Polyoxyethylen-Sorbitan-Fettsäureestern (Polysorbat), Natriumlaurylsulfat, Natriumstearylfumarat, Polyoxyethylenalkylether, Sorbitanfettsäureester, Polyethylenglykole (PEG),Polyoxyethylen Kastoröl-Derivaten, Docusatnatrium, quaternäre Ammoniumverbindungen, Aminosäuren wie L-Leucin, Zuckerester von Fettsäuren und Glyceriden von Fettsäuren ausgewählt wird, und im Bereich von vorzugsweise 0-10%, besser 0,1-5% nach Gewicht vorhanden ist.

15. Die Zusammensetzung nach irgendeinem der vorhergehenden Ansprüche, **dadurch gekennzeichnet, dass** Dosierungsform eine unten angegebene pharmazeutische Zusammensetzung aufweist:
NAC in einem Verhältnis bis zu 70% nach Gewicht
Natriumchlorid in einem Verhältnis bis zu 5% nach Gewicht
Zitronensäure-Anhydrid in einem Verhältnis von 0-85% nach Gewicht
Natriumhydrogencarbonat in einem Verhältnis von 0-85% nach Gewicht
Aspartam und/oder Zitronengeschmack in einem Verhältnis bis zu 5% nach Gewicht
PEG in einem Verhältnis von 0-10% nach Gewicht; und
PVP in einem Verhältnis von 0-10% nach Gewicht.

## Revendications

1. Une composition pharmaceutique **caractérisée en ce que** la composition comprend selon le poids noyau de la composition,
• NAC dans la plage de 900-2500 mg
• Chlorure de sodium à un taux jusqu'à 5% en poids
• Au moins un agent édulcorant et/ou agent de goût à un taux jusqu'à 5% en poids et
• Eventuellement un ou plus d'excipients pharmaceutiquement acceptable choisis entre l'agent de stabilisation, diluant, liant, désintégrant, lubrifiant, glissant et surfactant,
dans laquelle ladite composition est dans une forme de dosage d'un comprimé, capsule, comprimé à dissolution rapide ou bien comprimé effervescent.

2. La composition selon la revendication 1, **caractérisée en ce que**, les édulcorants sont choisis parmi la sucralose, la saccharose, le fructose, le glucose, le galactose, le xylose, la dextrose, le levulose, le lactose, le maltose, la maltodextrine, le mannitol, le maltitol, le maltol, le sorbitol, le xylitol, l'érythritol, le lactitol, l'isomalt, le sirop de mais, la saccharine, les sels de saccharine, l'acésulfame-potassium, l'aspartame, le D-tryptophane, le glyserrisate monoammonique, le néohespéridine dihydrochalcone, la thaumatine, le néotame, l'alitame, le stévioside et le cyclamate.

3. La composition selon la revendication 1, **caractérisé en ce que** les agents aromatisants sont choisis parmi les aromes naturels d'huile (e.g. l'huile de menthe, Partridge l'huile de cassis, l'huile de clou de girofle, l'huile de persil, l'huile d'eucalyptus, l'huile de citron, huile d'orange, etc.), le menthol, menta, l'anéthole, le salicylate de méthyle, l'eucalyptol, la cannelle, l'acétate de 1-méthyle, la sauge, l'eugénol, l'oxanone, l'alpha-irison, la marjolaine, le citron, l'orange, propényle guaethol acétyle, sinnamon, la vanille, le thymol, le linalol, cinnamaldehyde glycérol acétal, p-menthane- 3-carboxamide N-substitué, 3,1- propane diol-1,2 méthoxy.

4. La composition selon la revendication 1, **caractérisé en ce que**, le diluant est choisi parmi le lactose, la cellulose microcristalline, l'amidon, l'amidon pré-gélatinisé, l'amidon modifié, le phosphate de calcium (dibasique et/ou tribasique), le sulfate de calcium trihydraté, le sulfate de calcium dihydraté, le carbonate de calcium, le kaolin, le lactitol, la cellulose en poudre, le dextrose, les dextrates, la dextrine, le saccharose, le maltose, le fructose, le mannitol, le sorbitol et le xylitol.

5. La composition selon la revendication 4, **caractérisée en ce que** le diluant est de préférence dans la plage de 0-85%, de manière plus préférée 0.1-75% en poids.

6. La composition selon la revendication 1, **caractérisée en ce que** le liant est choisi parmi l'amidon (e.g. la fécule de pomme de terre, l'amidon de maïs, l'amidon de blé, etc.), le saccharose, le glucose, le dextrose, le lactose, les sucres tels que la maltodextrine, des gommes naturels et synthétiques (telles que la gomme arabique), la gélatine, les dérivés de cellulose (cellulose microcristalline, HPC, HEC, HPMC, la carboxyméthylcellulose, la méthylcellulose, l'éthylcellulose, etc.), la polyvinylpyrrolidone (PVP), le polyéthylène glycol (PEG), des cires, le carbonate de calcium, le phosphate de calcium, des alcools (e.g. le sorbitol, le xylitol, le mannitol) et de l'eau.

7. La composition selon la revendication 6, **caractérisée en ce que** le liant est de préférence dans la plage de 0-10%, de manière plus préférée 0.1-5% en poids.

8. La composition selon la revendication 1, **caractérisée en ce que**, les désintégrants sont choisi parmi l'amidon (l'amidon de maïs, la fécule de pomme de terre), le glycolate d'amidon sodique, l'amidon pré-gélatinisé, les dérivés de cellulose (e.g., la croscarmellose sodique ou la cellulose microcristalline), la polyvinylpyrrolidone (PVP), la crosspovidone, l'acide alginique, l'alginate de sodium, les argiles (la gomme de xanthane ou la Veegum etc.), des résines d'échange ionique et des systèmes effervescents (les carbonates de métaux alcalins ou alcalino-terreux [carbonate de sodium, le carbonate d'hydrogène de sodium, le carbonate de potassium, l'hydrogénocarbonate de potassium, de calcium carbonate, etc.], des acides solubles dans l'eau polyacides organiques et leurs sels tels que le sulfate d'hydrogène de sodium, de potassium hydrogénosulfate, le dihydrogénophosphate de sodium, l'acide succinique, l'acide tartrique, l'acide adipique, l'acide citrique.

9. La composition selon la revendication 8, **caractérisée en ce que** le désintégrant est de préférence dans la plage de 0-85%, de manière plus préférée 0.1-75% en poids.

10. La composition selon la revendication 1, **caractérisée en ce que**, le lubrifiant est choisi parmi les stéarates métalliques (le stéarate de magnésium, le stéarate de calcium, le stéarate d'aluminium, etc.), des esters d'acides gras (e.g. le sodium stéaryl fumarate), des acides gras (e.g., l'acide stéarique), des alcools gras, le béhénate de glycéryle, l'huile minérale, des paraffines, des huiles végétales hydrogénées, la leucine, les polyéthylèneglycols (PEG), des sulfates métalliques de lauryle (laurylsulfate de sodium, de magnésium du sulfate de lauryle, etc.), le chlorure de sodium, le benzoate de sodium, l'acétate de sodium et le talc.

11. La composition selon la revendication 10, **caractérisée en ce que** le lubrifiant est de préférence dans la plage de 0-10%, de manière plus préférée 0.1-5% en poids.

12. La composition selon la revendication 1, **caractérisée en ce que**, le glissant est choisi parmi le dioxyde de silicium, le trisilicate de magnésium, la cellulose en poudre, l'amidon, le talc, le phosphate de calcium tribasique, les stéarates métalliques, le silicate de calcium et le sulfate de lauryle métallique.

13. La composition selon la revendication 12, **caractérisée en ce que** le glissant est inférieur à 1% en poids.

14. La composition selon la revendication 1, **caractérisée en ce que** l'agent de surface est choisi parmi, les esters d'acides polyoxyéthlene-sorbitane (polysorbates), le laurylsulfate de sodium, le stéarylfumarate de sodium, des éthers alkyliques de polyoxyéthylène, les esters de sorbitan d'acides gras, les polyéthylèneglycols (PEG), dérivés de polyoxyéthylène d'huile de soupçon, le docusate de sodium, de composés d'ammonium quaternaire, les acides aminés tels que la L-leucine, les esters de sucres d'acides gras et les glycérides d'acides gras, et est présent de préférence dans la plage de 0-10%, de manière plus préférée 0.1-5% en poids.

15. La composition selon quelconque des revendications précédentes, **caractérisée en ce que**, la forme galénique comprend une composition pharmaceutique comme ci-dessous:
NAC à un taux jusqu'à 70% en poids
Chlorure de sodium à un taux jusqu'à 5% en poids
L'acide citrique anhydride à un taux de 0-85 % en poids
L'hydrogénocarbonate de sodium à un taux de 0-85 % en poids
La saveur d'aspartame et/ou de citron à un taux jusqu'à 5% en poids
PEG à un taux de 0-10% en poids; et
PVP à un taux de 0-10% en poids.
